# EUROPÄISCHE PATENTSCHRIFT

(11) **EP 3 941 359 B1**
(45) Veröffentlichungstag und Bekanntmachung des Hinweises auf die Patenterteilung: **12.07.2023**
(21) Anmeldenummer: 21703637.5
(22) Anmeldetag: 29.01.2021
(51) Int. Cl.: A61B 10/00, A61B 10/02

(54) **TUPFER ZUM AUFNEHMEN BIOLOGISCHER PROBEN**
SWAB FOR BIOLOGICAL SAMPLING
ÉCOUVILLON DE PRÉLÈVEMENT DE MATIÈRE BIOLOGIQUE

(30) Priorität: 14.05.2020 DE 102020113143; 25.06.2020 DE 102020116824
(43) Veröffentlichungstag der Anmeldung: 26.01.2022
(73) Patentinhaber: Vinceni Property II GmbH, 68163 Mannheim (DE)
(72) Erfinder: FÜTTERER, Ferdinand, 76532 Baden-Baden (DE)
(74) Vertreter: Reiser & Partner Patentanwälte mbB
(86) Internationale Anmeldenummer: PCT/EP2021/052150
(87) Internationale Veröffentlichungsnummer: WO 2021/228441

(56) Entgegenhaltungen:
- WO-A1-2019/237158
- DE-A1- 1 444 105
- DE-T2- 60 307 714
- US-A1- 2010 249 649
- US-A1- 2014 083 213
- US-A1- 2020 093 467

## Beschreibung

Die Erfindung betrifft einen Tupfer zum Aufnehmen biologischer Proben, umfassend ein Stäbchen, welches an einem freien Ende zumindest bereichsweise mit einer Flockfaseranordnung versehen ist.

Ein derartiger Tupfer ist beispielsweise aus der EP 1 608 268 B1 bekannt. Tupfer zum Aufnehmen biologischer Proben werde häufig verwendet, um biologische Proben, beispielsweise Sekret aus dem Nasen-, Mund- und Rachenraum, zu entnehmen und einer Untersuchung zuzuführen. Das Stäbchen ist dabei zumeist aus spritzgießfähigem Kunststoff ausgebildet, wobei die Länge so gewählt ist, dass beispielsweise Speichelproben aus dem Rachenraum entnommen werden können. Das Stäbchen bildet an einem freien Ende einen Handgriff aus und ist an dem anderen freien Ende mit einer Flockfaseranordnung versehen. Die Flockfasern sind dabei zumeist aus Kunststofffasern ausgebildet. Kunststofffasern sind vorteilhaft, weil sich diese biologisch neutral verhalten und das Untersuchungsergebnis nicht verfälschen. Besonders häufig werden in diesem Zusammenhang Polyamidfasern verwendet. Die Flockfasern sind über eine Klebeverbindung stoffschlüssig mit dem Stäbchen verbunden. Vor dem Beflocken wird auf das Stäbchen ein Kleberbett aufgetragen und die Flockfasern werden zum Aufbringen auf das Stäbchen einem elektrostatischen Feld ausgesetzt. Das führt dazu, dass sich die Flockfasern ausrichten, in das Kleberbett eintauchen und im Wesentlichen senkrecht aus der Oberfläche des Kleberbetts hervorstehen.

Bei den häufig verwendeten Kunststofffasern ist aber nachteilig, dass diese in der Regel hydrophob sind, also eine flüssigkeitsabweisende Oberfläche aufweisen. Damit der Tupfer dennoch ausreichend biologische Proben aufnehmen kann, werden die Flockfasern so dicht auf dem Stäbchen befestigt, dass sich zwischen den Stäbchen kapillaraktive Zwischenräume ergeben. Bei dem aus dem Stand der Technik bekannten Tupfer werden dabei Flockfasern mit einer einheitlichen Länge verwendet, welche wiederum eine einheitliche Schichtdicke auf dem Stäbchen bilden sollen. Es hat sich jedoch herausgestellt, dass die Anordnung der Flockfasern auf dem Stäbchen herstellungsbedingt Abweichungen von der senkrechten Ausrichtung zeigen, so dass die Flockfasern nicht parallel zueinander ausgerichtet sind. Dadurch verringern sich die kapillaraktiven Zwischenräume zwischen den Flockfasern.

Aus der US 2020/0093467 A1 ist ein Tupfer zum Aufnehmen biologischer Proben, bekannt, bei welchem in ersten Zonen Flockfasern erster Länge und in zweiten Zonen Flockfasern zweiter Länge vorgesehen sind. Die DE 1 444 105 A1 beschäftigt sich mit der Bereitstellung von Flockfasern.

Der Erfindung liegt die Aufgabe zugrunde, einen Tupfer zum Aufnehmen biologischer Proben bereitzustellen, welcher ein verbessertes Aufnahmevermögen aufweist. Diese Aufgabe wird mit den Merkmalen von Anspruch 1 gelöst. Auf vorteilhafte Ausgestaltungen nehmen die Unteransprüche Bezug.

Der erfindungsgemäße Tupfer zum Aufnehmen biologischer Proben umfasst ein Stäbchen, welches an einem freien Ende zumindest bereichsweise mit einer Flockfaseranordnung versehen ist, wobei die Flockfaseranordnung eine Flockfasermischung mit Flockfasertypen unterschiedlicher Länge umfasst.

Überraschenderweise hat sich herausgestellt, dass die Verwendung einer Flockfasermischung mit Flockfasertypen unterschiedlicher Länge eine Flockfaseranordnung ergibt, welche ein verbessertes Aufnahmevermögen für biologische Proben, insbesondere für flüssige biologische Proben wie Speichelproben, aufweist. Dies liegt daran, dass sich die Flockfasern herstellungsbedingt nicht ausschließlich senkrecht zur Oberfläche des Stäbchens ausrichten, sondern einzelne oder mehrere Fasern abweichend von der senkrechten Anordnung in dem Kleberbett des Stäbchens befestigt sind. Dies führt dazu, dass sich einzelne Flockfasern überkreuzen und die Fasern nicht ausschließlich parallel zueinander ausgerichtet sind. Durch die Verwendung von Flockfasern unterschiedlicher Länge ergeben sich jedoch trotz der Abweichung hinsichtlich der Ausrichtung der Fasern viele kapillaraktive Bereiche, so dass die Flockfaseranordnung ein hohes Aufnahmevermögen für biologische Proben aufweist.

Die Flockfaseranordnung kann zumindest zwei Flockfasertypen umfassen, wobei ein erster Flockfasertyp mindestens 0,3 mm, vorzugsweise mindestens 0,5 mm länger ist als ein zweiter Flockfasertyp. In Versuchen hat sich herausgestellt, dass sich bei einer derartigen Flockfaseranordnung ein besonders gutes Aufnahmevermögen für biologische Proben ergibt.

Die Flockfasertypen unterschiedlicher Länge können eine übereinstimmende Faserfeinheit aufweisen. Aufgrund der übereinstimmenden Faserfeinheit sind die kürzeren Fasern biegesteifer als die längeren Fasern. Somit weisen die kürzeren Fasern bei der Probenentnahme einen Borsteneffekt auf. Dadurch verbessern die härteren, kürzeren Fasern die Probenentnahme, während die weicheren, längeren Fasern das Speichervermögen für die entnommenen biologischen Proben verbessern.

Die Faserfeinheit der Flockfasertypen kann zwischen 1,7 dtex und 6,7 dtex, vorzugsweise 3,3 dtex betragen. Flockfasertypen mit einer derartigen Faserfeinheit sind bei der für den Tupfer in Frage kommenden Faserlänge aufgrund der Borstenwirkung und gleichzeitigen Weichheit besonders gut geeignet, biologische Proben aufzunehmen.

Der erste Flockfasertyp kann eine Länge von 1,5 mm und der zweite Flockfasertyp eine Länge von 1,0 mm aufweisen. Im Zusammenhang mit der zuvor genannten Faserfeinheit ergibt sich ein Stäbchen mit einem Flor, der besonders gut zum Aufnehmen biologischer Proben geeignet ist, wobei Irritationen während der Probenentnahme vermieden werden können. Dadurch, dass ein Flockfasertyp 0,5 mm länger ist als ein anderer Flockfasertyp, ergibt sich eine Mischung deutlich unterschiedlich langer Flockfasern. Dies ist insbesondere deswegen relevant, weil Flockfasern herstellungsbedingt eine Schnittlängentoleranz von 10% aufweisen können. Das bedeutet, dass Flockfasern des Flockfasertyps 1,0 mm eine Länge von 0,9 mm bis 1,1 mm und Flockfasertypen mit einer Länge von 1,5 mm eine Länge von 1,35 mm bis 1,65 mm aufweisen können.

Die Flockfasern der Flockfaseranordnung sind vorzugsweise aus Kunststoff, weiter vorzugsweise aus Polyamid ausgebildet. Flockfasern aus Polyamid haben sich im Zusammenhang mit Tupfern zum Aufnehmen biologischer Proben bewährt. Kunststoffflockfasern verhalten sich biologisch neutral, so dass Verfälschungen der Untersuchungsergebnisse vermieden werden können. Des Weiteren sind Kunststofffasern kostengünstig und in gleichbleibender Qualität herstellbar.

Die Flockfasern der Flockfaseranordnung können mit einem Aktivierungsmittel versehen sein, durch welches die Flockfasern elektrostatisch aufladbar sind. Dadurch, dass die Flockfasern in einem elektrostatischen Feld ausgerichtet und dann in ein Kleberbett eingeschossen werden sollen, ist es erforderlich, dass die Flockfasern elektrostatisch aufladbar sind. Durch Einwirkung eines elektrostatischen Feldes richten sich die Flockfasern aus und tauchen, beschleunigt durch das elektrostatische Feld, mit einem freien Ende in das Kleberbett ein. Das Aktivierungsmittel verbessert das elektrostatische Verhalten der Flockfasern, wodurch sich die Herstellbarkeit des Tupfers verbessert. Dabei ist das Aktivierungsmittel vorzugsweise ein Elektrolyt. Das Aktivierungsmittel wird dabei vorzugsweise in einer Flüssigkeit in Lösung gebracht und auf die Flockfasern aufgetragen.

Es hat sich gezeigt, dass das Aktivierungsmittel Einfluss auf das spätere Verhalten der Flockfaseranordnung hat und insbesondere das Aufnahmeverhalten für Flüssigkeiten beeinflusst. Hierbei hat sich herausgestellt, dass Elektrolyte, welche ausschließlich auf Natriumchlorid basieren, auf das spätere Aufnahmeverhalten nachteilige Auswirkungen haben können. Es hat sich gezeigt, dass ausschließlich mit Natriumchlorid ausgerüstete Flockfasern für Tupfer unzureichende hydrophile oder sogar hydrophobe Eigenschaften aufweisen können. Hingegen hat sich gezeigt, dass die Ausrüstung der Flockfasern mit einem Elektrolyten, enthaltend Natriumsulfat und/oder Natriumformiat in Verbindung mit einem Aktivator auf der Basis von Aluminiumsulfat, sowohl positive Auswirkungen auf das elektrostatische Verhalten hat als auch positive Auswirkungen auf das Aufnahmevermögen biologischer Proben aufweist. Mit einem derartigen Elektrolyten und Aktivator versehene Flockfasern sind hydrophil und weisen besonders gutes Aufnahmevermögen für biologische Proben auf.

Die Flockfaseranordnung ist vorzugsweise mittels eines Kleberbettes stoffschlüssig an dem Stäbchen befestigt. Durch das Kleberbett sind die Flockfasern sicher und dauerhaft an dem Stäbchen befestigt und lösen sich insbesondere bei der Probenentnahme nicht ab. Dabei ist das Kleberbett aus vorzugsweise aus lösemittelhaltigem Kleber ausgebildet. Im Unterschied zu einem wasserbasierenden Kleber weist ein lösemittelhaltiger Kleber flüchtige organische Komponenten auf. Des Weiteren ist der lösemittelhaltige Kleber vorzugsweise mit Katalysatoren versehen, welche eine Vernetzungsreaktion der Kleberbestandteile fördern.

Im Gegensatz zu einem wasserbasierenden Kleber ist die Topfzeit bei einem lösemittelhaltigen Kleber deutlich größer. Topfzeit ist dabei die Zeit, in der ein verarbeitungsfertiger Kleber in gleichbleibender Qualität verarbeitet werden kann. Dabei ist es insbesondere nicht erforderlich, den lösemittelhaltigen Kleber zur Aufrechterhaltung der Klebeeigenschaften während der Verarbeitung häufig aufzurühren. Ein weiterer Vorteil ist, dass mit Hilfe von Wärmezufuhr die Trockenzeit eines lösemittelbasierenden Klebers erheblich verkürzt werden kann. Durch die Wärmezufuhr verdampft das Lösemittel schneller und die Reaktionsfähigkeit der in dem lösemittelhaltigen Kleber enthaltenen Katalysatoren wird erhöht, wodurch sich wiederum die Vernetzung der auf dem Tupfer verbleibenden Kleberbestandteile beschleunigt. Die Haftung eines lösemittelhaltigen Klebers auf dem aus Kunststoff ausgebildeten Stäbchen ist besser als bei einem wasserbasierenden Kleber. Dabei ist die Haltekraft der aus Polyamid ausgebildeten Flockfasern durch den lösemittelhaltigen Kleber vergleichbar mit einem wasserbasierenden Kleber. Bei einem Abriebtest, bei welchem das mit Flockfasern beklebte Stäbchen einer Belastung durch Druck und Abrieb ausgesetzt wird, werden bei lösemittelhaltigem Kleber weniger Fasern herausgelöst als bei einem wasserbasierenden Kleber. Darüber hinaus ist der lösemittelhaltige Kleber unempfindlich gegenüber Feuchtigkeit und Nässe. Dies ist insbesondere in Bezug auf den Anwendungszweck einer biologischen Probenentnahme aus feuchtem Milieu durch den Tupfer vorteilhaft.

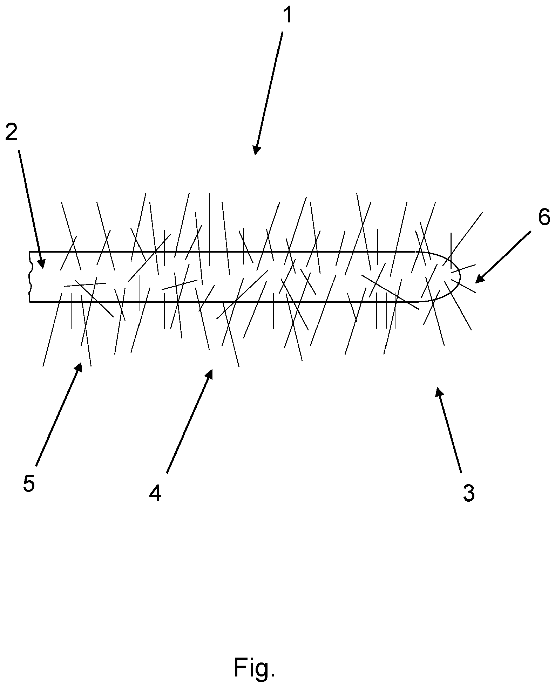

Eine Ausgestaltung des erfindungsgemäßen Tupfers wird nachfolgend anhand der Figur näher erläutert. Dies zeigt schematisch:
- Fig.: einen Tupfer im Bereich der Flockfaseranordnung.

Die Figur zeigt einen Tupfer 1 zum Aufnehmen biologischer Proben, umfassend ein Stäbchen 2 aus einem spritzgießfähigen, thermoplastischen Kunststoff, wobei je nach Verwendung insbesondere Polystyrol und Polyethylen Verwendung finden. Die Länge des Stäbchens ist so gewählt, dass biologische Proben aus der gewünschten Körperregion entnommen werden können. An einem freien Ende bildet das Stäbchen 2 einen Griffabschnitt aus, an dem anderen freien Ende 3 ist das Stäbchen 2 mit einer Flockfaseranordnung 4 versehen. Die Flockfaseranordnung 4 ist stoffschlüssig mittels einer Klebeverbindung an dem Stäbchen 2 fixiert.

Zum Herstellen des Tupfers 1 wird das Stäbchen 2 in einen Kleber getaucht, so dass sich an dem freien Ende 3 ein Kleberbett ausbildet. Bei der vorliegenden Ausgestaltung ist das Kleberbett aus lösemittelhaltigem Kleber ausgebildet, wobei das Lösemittel mit vernetzungsreaktionsfördernden Katalysatoren versehen ist. Danach werden Flockfasern mittels elektrostatischem Feld auf das Stäbchen 2 aufgebracht, wobei sich die Flockfasern aufgrund des Feldes ausrichten und mit einem Ende in das Kleberbett eintauchen. Anschließend wird das Stäbchen 2 mit der aufgeklebten Flockfaseranordnung 4 einer Wärmebehandlung unterzogen. Durch die Wärmebehandlung erfolgt eine Vernetzungsreaktion der Kleberbestandteile und eine Verkürzung der Herstellungsdauer. Die Flockfasern der Flockfaseranordnung 4 werden vor dem Aufbringen auf das Stäbchen 2 mit einem Aktivierungsmittel versehen, welches vorliegend eine Zusammensetzung ist. Die Zusammensetzung enthält als Elektrolyte Natriumsulfat und Natriumformiat und als Aktivator Aluminiumsulfat. Durch das Aktivierungsmittel verbessern sich sowohl die elektrostatischen als auch die hydrophilen Eigenschaften der Flockfasern.

Die Flockfaseranordnung 4 umfasst Flockfasertypen 5, 6 unterschiedlicher Länge. Bei der vorliegenden Ausgestaltung umfasst die Flockfaseranordnung 4 zwei Flockfasertypen 5, 6, wobei ein erster Flockfasertyp 5 0,5 mm länger ist als ein zweiter Flockfasertyp 6. Der erste Flockfasertyp 5 weist eine Länge von 1,5 mm und der zweite Flockfasertyp 6 eine Länge von 1,0 mm auf. Die Flockfasertypen 5, 6 unterschiedlicher Länge weisen mit jeweils 3,3 dtex eine übereinstimmende Faserfeinheit auf und bestehen aus Polyamid.

In der Flockfaseranordnung 4 beträgt der Mengenanteil des ersten Flockfasertypen 5 zwischen 20 Gew.% und 80 Gew.% und des zweiten Flockfasertypen 6 zwischen 20 Gew.% und 80 Gew.%. Eine besonders vorteilhafte Flockfaseranordnung 4 enthält 50 Gew.% des ersten Flockfasertypen 5 und 50 Gew.% des zweiten Flockfasertypen 6. Dabei sind fertigungsbedingt Schwankungen von jeweils 20 Gew.% möglich. Bei dieser Ausgestaltung weist die Flockfaseranordnung 4 ein besonders hohes Aufnahmevermögen für biologische Proben auf.

## Patentansprüche

1. Tupfer (1) zum Aufnehmen biologischer Proben, umfassend ein Stäbchen (2), welches an einem freien Ende (3) zumindest bereichsweise mit einer Flockfaseranordnung (4) versehen ist, **dadurch gekennzeichnet, dass** die Flockfaseranordnung (4) eine Flockfasermischung mit Flockfasertypen (5, 6) unterschiedlicher Länge umfasst.

2. Tupfer nach Anspruch 1, **dadurch gekennzeichnet, dass** die Flockfaseranordnung (4) zumindest zwei Flockfasertypen (5, 6) umfasst, wobei ein erster Flockfasertyp (5) mindestens 0,3 mm, vorzugsweise mindestens 0,5 mm länger ist als ein zweiter Flockfasertyp (6).

3. Tupfer nach Anspruch 1 oder 2, **dadurch gekennzeichnet, dass** die Flockfasertypen (5, 6) unterschiedlicher Länge eine übereinstimmende Faserfeinheit aufweisen.

4. Tupfer nach Anspruch 3, **dadurch gekennzeichnet, dass** die Faserfeinheit der Flockfasertypen (5, 6) zwischen 1,7 dtex und 6,7 dtex, vorzugsweise 3,3 dtex beträgt.

5. Tupfer nach einem der Ansprüche 2 bis 4, **dadurch gekennzeichnet, dass** der erste Flockfasertyp (5) eine Länge von 1,5 mm und der zweite Flockfasertyp (6) eine Länge von 1,0 mm aufweist.

6. Tupfer nach einem der Ansprüche 1 bis 5, **dadurch gekennzeichnet, dass** die Flockfasertypen (5, 6) aus Kunststoff, vorzugsweise aus Polyamid ausgebildet sind.

7. Tupfer nach einem der Ansprüche 1 bis 6, **dadurch gekennzeichnet, dass** die Flockfasertypen (5, 6) mit einem Aktivierungsmittel versehen sind, durch welches die Flockfasertypen (5, 6) elektrostatisch aufladbar sind.

8. Tupfer nach Anspruch 7, **dadurch gekennzeichnet, dass** das Aktivierungsmittel ein Elektrolyt ist.

9. Tupfer nach einem der Ansprüche 1 bis 8, **dadurch gekennzeichnet, dass** die Flockfaseranordnung (4) mittels eines Kleberbettes stoffschlüssig an dem Stäbchen (2) befestigt ist.

10. Tupfer nach Anspruch 9, **dadurch gekennzeichnet, dass** das Kleberbett aus lösemittelhaltigem Kleber ausgebildet ist.

## Claims

1. Swab (1) for collecting biological samples, comprising a small stick (2) that is provided on a free end (3) at least in regions with a flock fibre arrangement (4), **characterised in that** the flock fibre arrangement (4) comprises a flock fibre mixture having flock fibre types (5, 6) of different length.

2. Swab according to claim 1, **characterised in that** the flock fibre arrangement (4) comprises at least two flock fibre types (5, 6), wherein a first flock fibre type (5) is at least 0.3 mm, preferably at least 0.5 mm, longer than a second flock fibre type (6).

3. Swab according to claim 1 or 2, **characterised in that** the flock fibre types (5, 6) of different length comprise a matching fibre fineness.

4. Swab according to claim 3, **characterised in that** the fibre fineness of the flock fibre types (5, 6) is between 1.7 dtex and 6.7 dtex, preferably 3.3 dtex.

5. Swab according to one of claims 2 to 4, **characterised in that** the first flock fibre type (5) comprises a length of 1.5 mm and the second flock fibre type (6) comprises a length of 1.0 mm.

6. Swab according to one of claims 1 to 5, **characterised in that** the flock fibre types (5, 6) are embodied from synthetic material, preferably from polyamide.

7. Swab according to one of claims 1 to 6, **characterised in that** the flock fibre types (5, 6) are provided with an activation means by means of which the flock fibre types (5, 6) can be electrostatically charged.

8. Swab according to claim 7, **characterised in that** the activation means is an electrolyte.

9. Swab according to one of claims 1 to 8, **characterised in that** the flock fibre arrangement (4) is fastened by means of an adhesive bed in a materially-bonded manner to the small stick (2).

10. Swab according to claim 9, **characterised in that** the adhesive bed is embodied from solvent-based adhesive.

## Revendications

1. Écouvillon (1) de prélèvement d'échantillons biologiques, comprenant un bâtonnet (2) qui est muni à une extrémité libre (3), au moins partiellement, d'un ensemble de fibres de flocage (4), **caractérisé en ce que** l'ensemble de fibres de flocage (4) comprend un mélange de fibres de flocage avec des types de fibres de flocage (5, 6) de différentes longueurs.

2. Écouvillon selon la revendication 1, **caractérisé en ce que** l'ensemble de fibres de flocage (4) comprend au moins deux types de fibres de flocage (5, 6), dans lequel un premier type de fibres de flocage (5) est plus long d'au moins 0,3 mm, de préférence d'au moins 0,5 mm, qu'un second type de fibres de flocage (6).

3. Écouvillon selon la revendication 1 ou 2, **caractérisé en ce que** les types de fibres de flocage (5, 6) de différentes longueurs ont une finesse de fibre correspondante.

4. Écouvillon selon la revendication 3, **caractérisé en ce que** la finesse de fibre des types de fibres de flocage (5, 6) est comprise entre 1,7 dtex et 6,7 dtex, de préférence de 3,3 dtex.

5. Écouvillon selon l'une des revendications 2 à 4, **caractérisé en ce que** le premier type de fibres de flocage (5) a une longueur de 1,5 mm et le second type de fibres de flocage (6) a une longueur de 1,0 mm.

6. Écouvillon selon l'une des revendications 1 à 5, **caractérisé en ce que** les types de fibres de flocage (5, 6) sont réalisés en matière plastique, de préférence en polyam ide.

7. Écouvillon selon l'une des revendications 1 à 6, **caractérisé en ce que** les types de fibres de flocage (5, 6) sont munis d'un agent d'activation au moyen duquel les types de fibres de flocage (5, 6) peuvent être chargés électrostatiquement.

8. Écouvillon selon la revendication 7, **caractérisé en ce que** l'agent d'activation est un électrolyte.

9. Écouvillon selon l'une des revendications 1 à 8, **caractérisé en ce que** l'ensemble de fibres de flocage (4) est fixé sur le bâtonnet (2) par liaison de matière au moyen d'un lit de colle.

10. Écouvillon selon la revendication 9, **caractérisé en ce que** le lit de colle est réalisé à partir d'une colle contenant du solvant.
